# FASCICULE DE BREVET EUROPEEN

(11) **EP 0 507 650 B1**
(45) Date de publication et mention de la délivrance du brevet: **22.05.1996**
(21) Numéro de dépôt: 92400780.0
(22) Date de dépôt: 23.03.1992
(51) Int. Cl.: C07D 401/14, A61K 31/415, C07D 417/14, C07D 413/14, A61K 31/445

(54) **Dérivés de pipéridine, leur préparation et leur application en thérapeutique**
Piperidinderivate, Verfahren zu deren Herstellung und deren therapeutische Anwendung
Piperidine derivatives, their preparation and their therapeutic application

(30) Priorité: 03.04.1991 FR 9104009
(43) Date de publication de la demande: 07.10.1992
(73) Titulaire: SYNTHELABO, F-92350 Le Plessis Robinson (FR)
(72) Inventeur: Jegham, Samir, F-95100 Argenteuil (FR); Defosse, Gérard, F-75013 Paris (FR); Purcell, Thomas, F-78490 Montfort-L'Amaury (FR); Schoemaker, Johannes, F-91190 Gif-sur-Yvette (FR)
(74) Mandataire: Thouret-Lemaitre, Elisabeth

(56) Documents cités:
- EP-A- 0 197 840
- EP-A- 0 445 026

## Description

La présente invention a pour objet des dérivés de pipéridine, leur préparation et leur application en thérapeutique.

Les composés de l'invention répondent à la formule générale (I) du schéma de l'annexe, dans laquelle
R₁ représente soit un atome d'hydrogène, soit un groupe (C₁₋₆)alkyle droit ou ramifié, soit un groupe cyclo(C₃₋₈)alkyle, X représente soit un atome d'oxygène, soit un atome de soufre soit un groupe de formule générale N-R₃ dans laquelle R₃ est un atome d'hydrogène, un groupe (C₁₋₈)alkyle droit ou ramifié, cyclo(C₃₋₆)alkyle, cyclo(C₃₋₆)alkylméthyle, (C₁₋₄)alcoxy(C₁₋₄)alkyle, phényle, pyridin-4-yle, pyridin-3-yle,
Z représente un atome d'hydrogène ou de fluor.

Les composés préférés, selon l'invention, sont les composés de formule générale (I) dans laquelle
R₁ représente soit un atome d'hydrogène, soit un groupe (C₁₋₆)alkyle droit ou ramifié,
X représente un groupe de formule générale N-R₃ dans laquelle R₃ est un groupe (C₁₋₈)alkyle droit ou ramifié,
Z représente un atome d'hydrogène.

Enfin les composés de choix sont ceux répondant à la formule générale (I) dans laquelle
R₁ représente soit un atome d'hydrogène, soit un groupe méthyle,
X représente un groupe (1-méthyléthyl)imino,
Z représente un atome d'hydrogène.

Ils peuvent se présenter à l'état de bases libres ou de sels d'addition à des acides pharmaceutiquement acceptables. Les composés dont la formule est une forme mésomère de la formule (I), font partie de l'invention.

Conformément à l'invention, on peut préparer les composés de formule générale (I) selon le procédé illustré dans le schéma de l'annexe.

On fait réagir un dérivé de pyridine de formule générale (IX), dans laquelle R₁ est tel que défini ci-dessus, avec du chlorhydrate d'hydroxylamine, en présence de soude concentrée dans un solvant tel que l'eau. On obtient un composé de formule générale (VIII) dans lequel on crée un groupe partant, à partir du groupe hydroxyle, en le soumettant par exemple à l'action du chlorure de tosyle dans un solvant tel que la pyridine, pour obtenir un composé de formule générale (VII). Ce composé, en présence d'une base telle que, par exemple, l'éthylate de potassium dans l'éthanol absolu, réagit pour donner un intermédiaire cyclique instable. On hydrolyse cet intermédiaire, par exemple au moyen d'une solution d'acide chlorhydrique concentré dans l'eau, et on obtient un composé de formule générale (VI). L'action du thiocyanate de potassium sur ce composé provoque la formation d'un cycle imidazole portant un groupe mercapto [composé (V)], que l'on élimine par réaction avec une solution oxydante, telle que par exemple une solution aqueuse d'acide nitrique. On obtient ainsi un composé de formule générale (IV), dont on hydrogène le noyau pyridinique par hydrogénation catalytique. On obtient un dérivé de pipéridine de formule générale (III), sur lequel on fait réagir finalement un dérivé chloré de formule générale (II).

Les composés de départ sont décrits dans la littérature ou peuvent être préparés selon des méthodes qui y sont décrites ou qui sont connues de l'homme de métier.
Les composés de formule générale (IX) dans laquelle R₁ représente un atome d'hydrogène sont disponibles dans le commerce. Ceux pour lesquels R₁ est autre qu'un atome d'hydrogène sont préparés à partir de la 4-cyanopyridine par action de l'organomagnésien R₁-CH₂-Mg-Hal correspondant où Hal est Br ou Cl, suivie d'une hydrolyse acide.
Des dérivés chlorés de formule générale (II) dans laquelle X représente un atome d'oxygène sont décrits dans *J. Med. Chem.* , 1988, **31**, 1719-28. Ceux dans la formule desquels X représente un groupe N-phényle sont accessibles à partir de la *N*-phénylbenzène-1,2-diamine, d'abord par réaction avec l'urée pour former la 1-phénylbenzimidazolone, puis par réaction avec le chlorure de phosphoryle.
Les composés de formule générale (II), dans laquelle X représente un groupe NR₃, et R₃ est tel que défini précédemment, ont été préparés suivant des protocoles voisins de ceux décrits dans *J. Med. Chem.*, 1986, **29**, 1178-83 et dans le brevet européen n° 0039190.

La 4-(5-méthyl-1*H*-imidazol-4-yl)pyridine est décrite dans *J. Med. Chem.*, 1986, **29**, 2154-63.

Le 1-(pyridin-4-yl)éthanone-oxime et le 1-(pyridin-4-yl)éthanone-*O*-[(4-méthylphényl)sulfonyl]oxime sont décrits dans *Org. Synth*., 1985, **64**, 19-26.

La 4-(1*H*-imidazol-4-yl)pipéridine est décrite dans *Arch*. *Pharmaz.*, (Weinheim. Ger.) 1973, **306**(12), 934-42 et dans la demande de brevet européen 0197840.

Les exemples 1 à 2 qui suivent illustrent la préparation de quelques composés de formule (II).
Les exemples 3 et 4 qui suivent illustrent en détail la préparation des composés selon l'invention.
Les microanalyses et les spectres IR et RMN confirment la structure des composés obtenus.

### Exemple 1

### 2-chloro-5-fluoro-1(1-méthyléthyl)-1H-benzimidazole.

### 1.1. 4-fluoro-N-(1-méthyléthyl)-2-nitrobenzènamine.

On introduit dans un ballon 25 g (0,157 mole) de 2,5-difluoro-nitrobenzène, et 40,3 ml (0,47 mole) de 1-méthyléthylamine. La réaction est exothermique. On laisse le mélange au repos pendant une nuit. On ajoute ensuite 500 ml d'eau, on essore le précipité qui s'est formé, on le lave à l'eau et on le sèche sous vide sur pentoxyde de phosphore. On obtient 31 g de composé.
Point de fusion : 48-50°C.

### 1.2. 5-fluoro-1-(1-méthyléthyl)-1H-benzimidazol-2(3H)-one.

On introduit, dans un appareil de Parr, 31 g (0,156 mole) du composé précédent en solution dans 200 ml de méthanol, et une quantité catalytique de nickel de Raney. On effectue l'hydrogénation pendant 1,5 heures, à 50°C à une pression de 0,35 MPa. On filtre ensuite le nickel. La solution incolore devient brunâtre au contact de l'air. On la concentre. On ajoute 12,4 g (0,207 mole) d'urée. On maintient à 180°C pendant 3 heures. On obtient alors un magma solide, que l'on reprend par un peu d'eau chaude. On essore le précipité qui s'est formé, et on le recristallise dans l'éthanol. On obtient 17 g de composé.
Point de fusion : 156°C.

### 1.3. 2-chloro-5-fluoro-1-(1-méthyléthyl)-1H-benzimidazole.

On ajoute à 16 g (0,082 mole) du composé précédent 140 ml de chlorure de phosphoryle. On porte ce mélange au reflux pendant 3 heures. Puis on évapore le solvant, on ajoute de l'eau et on alcalinise à l'aide de soude. On extrait alors le mélange à l'éther, on le sèche, et on le purifie sur colonne de gel de silice, en éluant avec un mélange d'acétate d'éthyle et d'hexane, dans les proportions 20/80 en volume. On récupère 12 g de composé pur.
Point de fusion : 60°C.

### Exemple 2

### 2-chloro-1-(1-méthyléthyl)-1H-benzimidazole.

On introduit dans un ballon 13,5 g (0,0885 mole) de 2-chloro-1*H*-benzimidazole, 9,2 ml de 1-bromo-1-méthyléthyle, 230 g de carbonate de potassium et 200 ml de diméthylsulfoxyde. On agite vigoureusement pendant 4 heures en maintenant la température à 60°C. On extrait plusieurs fois à l'éther. On réunit les phases organiques et on concentre. On purifie le résidu pâteux sur colonne de gel de silice en éluant avec un mélange d'acétate d'éthyle et d'hexane dans les proportions 20:80 v/v. On concentre les fractions pures sous vide. On dissout le résidu dans 20 ml d'hexane à chaud. On refroidit à l'aide d'un bain de glace, on essore les cristaux puis on les lave avec un peu d'hexane, et on sèche sous vide. On obtient 12 g de composé.
Point de fusion : 62°C.

### Exemple 3

2-[4-(5-Méthyl-1*H*-imidazol-4-yl)pipéridin-1-yl]-1-(1-méthyléthyl)-1*H*-benzimidazole.

### 3.1. 1-(Pyridin-4-yl)propan-1-one oxime.

A une solution de 79 g (1,13 moles) de chlorhydrate d'hydroxylamine dans 1 litre d'eau refroidie au bain de glace, on ajoute 113 ml (1,13 moles) de soude 10 N puis, en 5 minutes, 107 g (0,79 mole) de 1-(pyridin-4-yl)propan-1-one, préparée à partir de la 4-cyanopyridine suivant le protocole décrit dans le brevet WO 85/02402.
On ajoute à cette solution 300 ml de méthanol et on porte au reflux pendant 2 heures. On refroidit la solution, qui précipite. On essore le précipité puis on le sèche sous vide sur pentoxyde de phosphore. On obtient 96 g de composé.
Point de fusion : 152°C.

### 3.2. 1-(Pyridin-4-yl)propan-1-one-O-[(4-méthylphényl)sulfonyl]oxime.

A une solution de 142 g (0,75 mole) de chlorure de tosyle dans 500 ml de pyridine, on ajoute 96 g (0,65 mole) du composé précédent. On agite pendant 24 heures, puis on jette la solution sur de l'eau glacée, on essore le précipité formé, on le lave à l'eau puis on le sèche et on le recristallise dans 300 ml d'alcool isopropylique. On obtient 120 g de composé.
Point de fusion : 90°C.

### 3.3. Chlorhydrate de 2-amino-1-(pyridin-4-yl)propan-1-one(2:1).

Dans un réacteur de 2 litres, on place 28 g (0,32 mole) d'éthylate de potassium dans 250 ml d'éthanol absolu, sous atmosphère d'argon. On refroidit cette solution à l'aide d'un bain de glace puis on y additionne une solution tiède de 79 g (0,26 mole) du composé précédent dans 400 ml d'éthanol. On agite pendant 3 heures à température ambiante, on ajoute ensuite 1 litre d'éther et on filtre le précipité de tosylate de potassium. On verse lentement et sous atmosphère d'argon la solution organique restante sur 400 ml (0,8 mole) d'acide chlorhydrique 2 N. On décante et on lave la phase organique avec 100 ml d'acide chlorhydrique 2 N. Puis, on réunit les phases aqueuses et on les concentre sous vide à une température inférieure ou égale à 40°C. Le résidu est repris dans un mélange éthanol/toluène en proportions 50:50 v/v. La suspension résultante est évaporée sous vide pour éliminer les traces d'eau. Le résidu cristallisé est repris par 50 ml d'éthanol chaud. On refroidit cette solution, on la filtre, on la lave à l'éthanol puis on la sèche sous vide sur pentoxyde de phosphore. On obtient 49 g de composé.
Point de fusion : 210°C.

### 3.4. 4-(2-Mercapto-5-méthyl-1H-imidazol-4-yl)pyridine.

A une solution de 49,7 g (0,22 mole) du composé précédent dans 210 ml d'eau, on ajoute 25,6 g (0,26 mole) de thiocyanate de potassium, puis on chauffe à 100°C à l'aide d'un bain d'huile, pendant 1 heure. On refroidit ensuite la solution à 30°C, on filtre le précipité, on le lave à l'eau puis on le sèche, et on l'additionne, sans agiter, à une solution de bicarbonate de sodium. On essore le précipité, on le lave à l'eau et on le sèche. On obtient 33 g de composé. Point de fusion : > 290°C.

### 3.5. 4-(5-Méthyl-1H-imidazol-4-yl)pyridine.

Dans un réacteur de 6 litres, on place 33 g (0,172 mole) du composé précédent en présence de 255 ml d'acide nitrique (d = 1,41) et de 1,4 litres d'eau. On chauffe à 85°C en agitant et la réaction d'oxydation se fait violemment avec libération de vapeurs sulfureuses piégées par de la soude diluée. La température de la réaction s'élève à 92°C, puis est ensuite réglée à 100°C pendant 1 heure. On refroidit la solution à l'aide d'un bain de glace, puis on alcalinise avec du bicarbonate de sodium. On filtre, on lave les sels minéraux trois fois avec 300 ml d'éthanol, puis on évapore le filtrat alcoolique. Le résidu solide est recristallisé dans l'eau. On l'essore, on le lave avec un minimum d'eau, puis on le sèche sous vide. On obtient 22 g de composé.
Point de fusion : 185°C.

### 3.6. Chlorhydrate de 4-(5-méthyl-1H-imidazol-4-yl)pipéridine (2 : 1).

On introduit dans un appareil de Parr 22 g (0,138 mole) du composé précédent, 80 ml d'acide chlorhydrique concentré, 500 ml d'eau et 3 g de charbon rhodié à 5 %. On agite le mélange à 35°C sous une pression de 0,35 MPa. Après 4 heures, l'hydrogénation est terminée ; on filtre le catalyseur et on évapore le filtrat sous vide. Le résidu est repris par un mélange d'éthanol et de toluène. La suspension résultante est évaporée sous vide pour chasser les traces d'eau. Le résidu solide est repris par 50 ml d'éthanol chaud. On refroidit cette solution, on essore le précipité, on le lave à l'éthanol, et on le sèche sous vide sur pentoxyde de phosphore. On obtient 26 g de composé.
Point de fusion : 320°C.

### 3.7. 2-[4-(5-Méthyl-1H-imidazol-4-yl)pipéridin-1-yl]-1-(1-méthyléthyl)-1H-benzimidazole.

A une supension de 21,5 g (0,0904 mole) du composé précédent dans 100 ml de méthanol, on ajoute 34 ml (0,18 mole) de méthylate de sodium 5,3 N, sous argon. On agite pendant 15 minutes, puis on concentre sous vide aux deux tiers. On ajoute ensuite 200 ml de dichlorométhane, et on filtre le chlorure de sodium. On concentre la solution sous vide et on récupère la base cristallisée. On ajoute ensuite une solution de 400 ml de 3-méthylbutan-1-ol et de 8,8 g (0,0452 mole) de 2-chloro-1(1-méthyléthyl)-1*H*-benzimidazole.
On chauffe à 120°C à l'aide d'un bain d'huile pendant 24 heures. On filtre le précipité de chlorhydrate de pipéridine (1:1) en excès (6 g). Le filtrat est évaporé à sec et le résidu est purifié sur colonne de gel de silice en éluant avec un mélange de dichlorométhane et de méthanol en proportions 90:10 v/v. On réunit les fractions pures, on évapore. On lave le solide à l'acétone, puis on sèche sous vide. On obtient 9,5 g de composé.
Point de fusion : 258°C.

### Exemple 4

2-[4-(1*H*-imidazol-4-yl)pipéridin-1-yl]-1-(1-méthyléthyl)-1*H*-benzimidazole.

### 4.1. 1-(Pyridin-4-yl)éthanone-oxime.

Dans un ballon, on ajoute une solution de 139 g (2 moles) de chlorhydrate d'hydroxylamine dans 280 ml d'eau à une solution de 200 ml (2 moles) de soude 10 N dans 180 ml d'eau, en maintenant la température vers -5°C. On ajoute rapidement 200 g (1,65 moles) de 1-(pyridin-4-yl)éthanone et on continue l'agitation pendant 2 heures à 0°C. On filtre le précipité formé, et on le lave plusieurs fois à l'eau glacée. On recristallise dans 4 litres d'eau chaude, et on laisse refroidir la solution pendant la nuit. On essore les cristaux, on lave à l'eau, on sèche à l'étuve sur pentoxyde de phosphore. On obtient 148 g de composé.
Point de fusion : 153°C.

### 4.2. 1-(Pyridin-4-yl)éthanone-O-[(4-méthylphényl)sulfonyl]oxime.

On ajoute 148 g (1,09 moles) du composé précédent à une solution de 261 g (1,37 moles) de chlorure de tosyle dans 550 ml de pyridine, refroidie à 0°C. On laisse revenir le mélange réactionnel à température ambiante et on agite pendant 48 heures. On verse ensuite la solution sur 3 litres d'eau glacée, on essore le précipité qui s'est formé, on le lave à l'eau puis on le sèche sous vide sur pentoxyde de phosphore. On recristallise dans 600 ml d'alcool isopropylique, on essore, on lave à l'hexane et on sèche sous vide. On obtient 275 g de composé.
Point de fusion : 80°C.

### 4.3. Chlorhydrate de 2-amino-1-(pyridin-4-yl)éthanone (2:1).

Dans un réacteur de 6 litres contenant 500 ml d'éthanol absolu, on ajoute 100 g (1,19 moles) d'éthylate de potassium en poudre, à 0°C, sous atmosphère d'argon. On additionne ensuite une solution tiède de 295 g (1,017 moles) du composé précédent dans 1,5 litres d'éthanol absolu, de façon à ce que la température de réaction n'excède pas 0°C. On laisse ensuite revenir à température ambiante et on agite encore 1 heure. On ajoute ensuite 4 litres d'éther anhydre, et on filtre le précipité de tosylate de potassium. On ajoute lentement et sous atmosphère d'argon le filtrat organique à une solution de 350 ml (2,1 moles) d'acide chlorhydrique 6 N dans 800 ml d'eau. On décante, on relave la phase organique avec 200 ml d'acide chlorhydrique 2 N, on réunit les phases aqueuses et on les concentre sous vide à une température inférieure ou égale à 40°C. Le résidu cristallisé est repris par 200 ml d'éthanol chaud. On refroidit, on filtre, on lave à l'éthanol puis on sèche sous vide le précipité. On obtient 200 g de composé.
Point de fusion : 230°C.

### 4.4. 4-(2-Mercapto-1H-imidazol-4-yl)pyridine.

On chauffe à 110°C à l'aide d'un bain d'huile, pendant 1,5 heures, un mélange de 200 g (0,956 mole) du composé précédent, de 92,8 g (0,956 mole) de thiocyanate de potassium, et de 800 ml d'eau. On laisse refroidir le mélange vers 40°C, on filtre le précipité formé et on le lave à l'eau. On l'ajoute ensuite par petites portions à 80 g (0,95 mole) de bicarbonate de sodium dans 500 ml d'eau. On filtre le précipité, on le lave à l'eau, et on le sèche sous vide. On obtient 66,5 g de composé.
Point de fusion : > 290°C.

### 4.5. 4-(1H-Imidazol-4-yl)pyridine.

Dans un réacteur de 10 litres, on introduit 66 g (0,372 mole) du composé précédent dans une solution de 550 ml d'acide nitrique (d = 1,41) et de 3,2 litres d'eau. On chauffe à 90°C en agitant bien, et la réaction se fait violemment, avec libération de vapeurs sulfureuses piégées par de la soude diluée. La température de réaction s'élève à 95°C et est ensuite réglée à 100°C pendant 1 heure. Puis on refroidit à - 7°C : un précipité se forme ; on l'essore, on le lave avec un peu d'eau glacée. Le précipité humide est repris ensuite avec 130 ml d'eau chaude. Puis on ajoute, par petites portions, 72 g de bicarbonate de sodium en poudre, ce qui provoque une cristallisation. On refroidit à l'aide d'un bain de glace, on essore les cristaux et on les sèche sous vide ; on les reprend plusieurs fois par une solution de dichlorométhane et de méthanol (90:10 v/v) chaude afin d'éliminer les sels minéraux. Le filtrat est concentré et l'on obtient 42,4 g de composé pur.
Point de fusion : 185°C.

### 4.6. Chlorhydrate de 4-(1H-imidazol-4-yl)pipéridine (2:1).

Dans un appareil de Parr, on introduit 42 g (0,289 mole) du composé précédent en solution dans 156 ml d'acide chlorhydrique concentré et 900 ml d'eau, en présence de rhodium à 5 % sur charbon. On effectue l'hydrogénation à 35°C, sous une pression de 0,35 MPa. Après 4 heures d'agitation, la réduction est terminée. On filtre le catalyseur et on évapore le filtrat. Le résidu est repris par un mélange d'éthanol et de toluène dans les proportions 50:50 v/v. La suspension résultante est évaporée sous vide, pour chasser les traces d'eau. Le résidu solide est ensuite repris par 100 ml d'éthanol chaud. On refroidit, on essore, on lave à l'éthanol puis on sèche sous vide sur pentoxyde de phosphore. On obtient 61 g de composé.
Point de fusion : 290°C.

### 4.7. 2-[4-(1H-imidazol-4-yl)pipéridin-1-yl]-1-(1-méthyléthyl)-1H-benzimidazole.

On ajoute à une solution de 27,6 g (0,123 mole) du composé précédent, 47,3 ml (0,25 mole) de méthylate de sodium. On agite pendant 15 minutes, et on concentre sous vide aux deux tiers, et on ajoute 200 ml de dichlorométhane. Il se forme un précipité de chlorure de sodium que l'on filtre. On concentre le filtrat sous vide. Le résidu cristallisé est mis à réagir avec 12 g (0,061 mole) de 2-chloro-1-(1-méthyléthyl)-1*H*-benzimidazole dans 60 ml de 3-méthylbutan-1-ol à 120°C pendant 36 heures. On filtre le précipité de monochlorhydrate de pipéridine en excès (8 g). Le filtrat est évaporé à sec. Le résidu obtenu est purifié sur colonne de gel de silice en éluant avec un mélange de dichlorométhane et de méthanol en proportions variables de 95:5 à 90:10 v/v. On réunit les fractions pures, on évapore. On lave le solide à l'éther et on sèche sous vide. On obtient 14,3 g de composé.
Point de fusion : 183°C.

Le tableau suivant illustre les structures chimiques et les propriétés physiques de quelques composés selon l'invention.

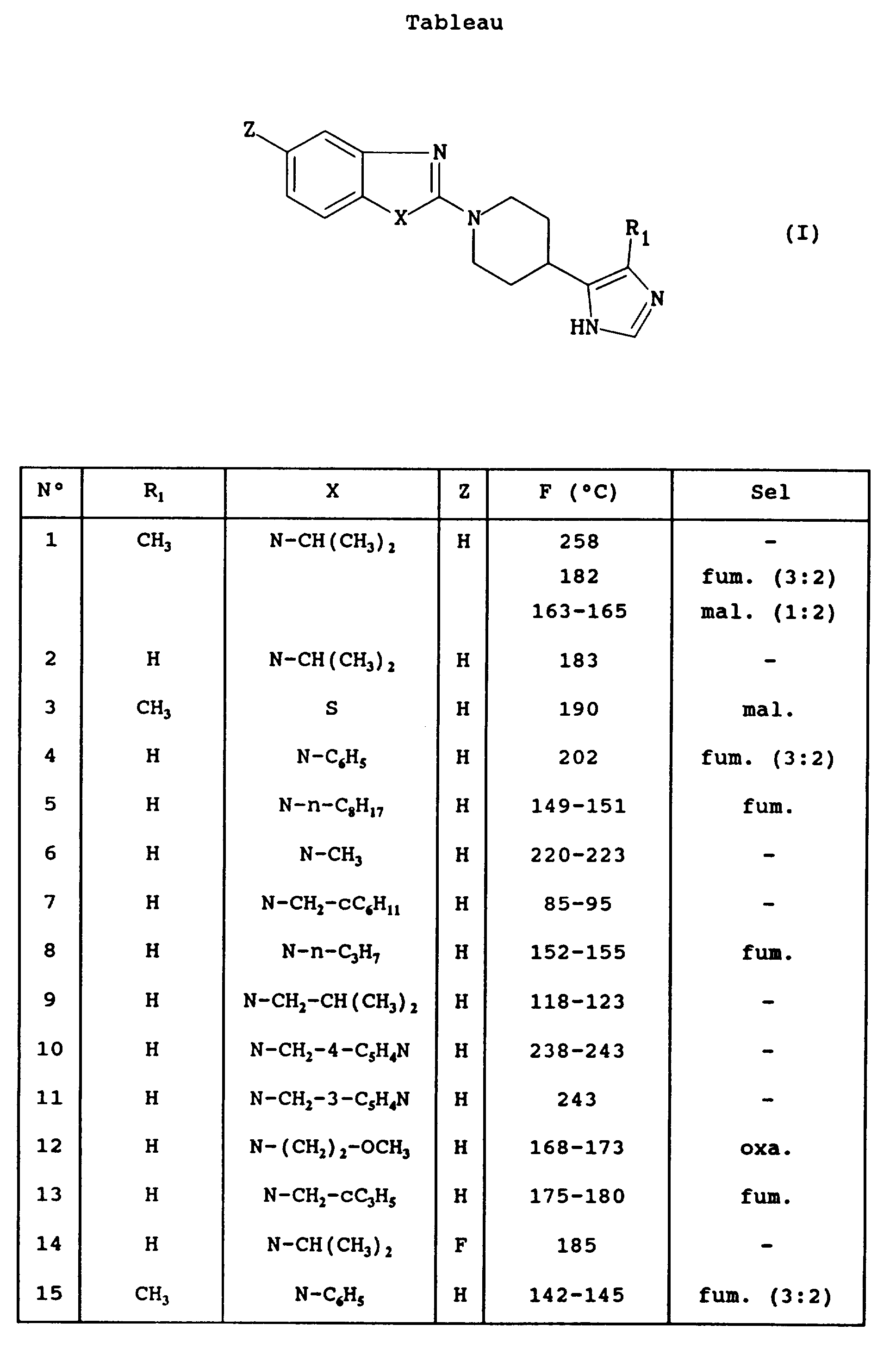

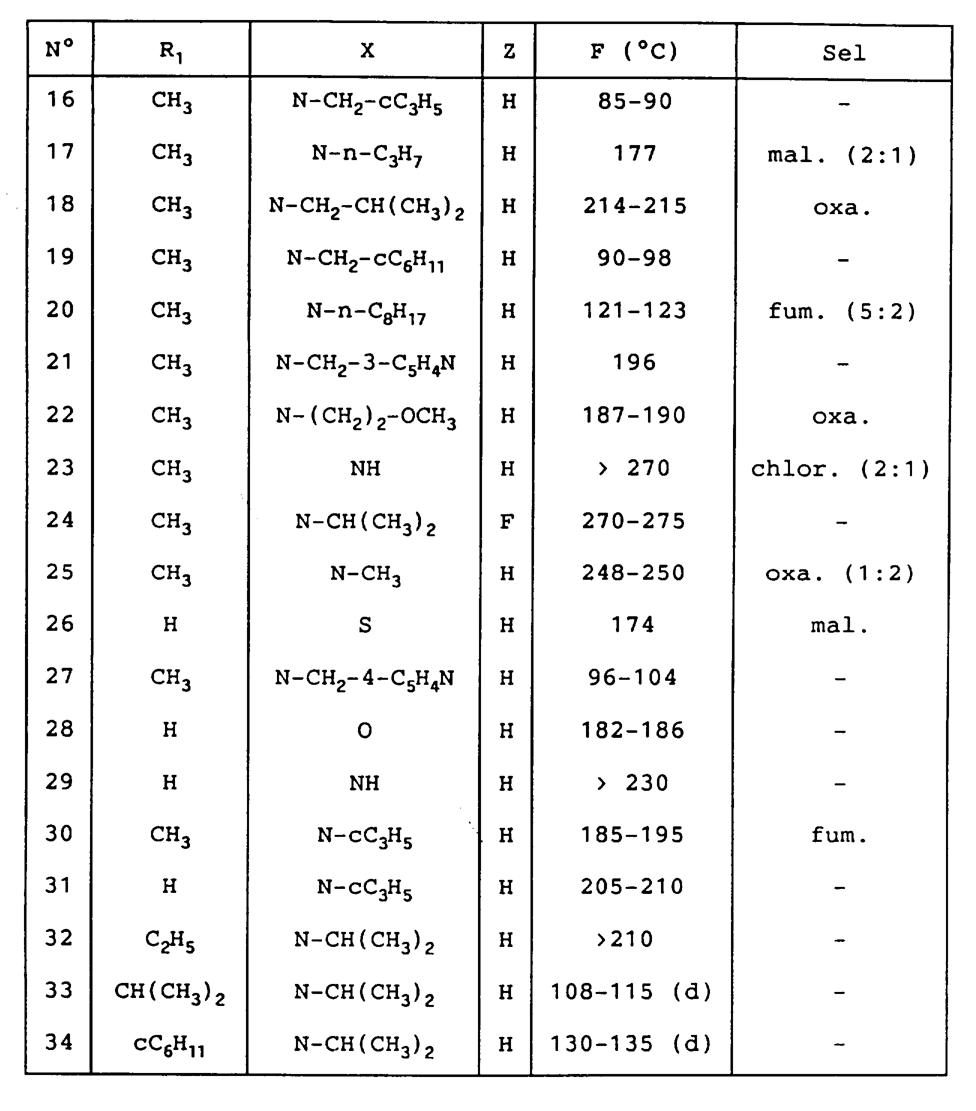

NB :dans la colonne "X" du tableau
cC₆H₁₁ signifie cyclohexyle,
4-C₅H₄N signifie pyridin-4-yle,
3-C₅H₄N signifie pyridin-3-yle,
cC₃H₅ signifie cyclopropyle;
dans la colonne "F (°C)" du tableau
(d) signifie décomposition
dans la colonne "Sel" du tableau
(x:y) signifie x moles d'acide pour y moles de base, l'absence de toute mention signifie que le composé est à l'état de base,
chlor. représente le chlorhydrate,
fum. représente le fumarate,
mal. représente le maléate,
oxa. représente l'oxalate.

Les composés de l'invention ont fait l'objet d'essais pharmacologiques qui ont montré leur intérêt comme substances actives en thérapeutique.

Ainsi ils ont été testés quant à leurs effets inhibiteurs de la liaison de la [³H]quipazine avec les récepteurs sérotoninergiques de type 5-HT₃ présents dans le cortex cérébral du rat, selon une variante de la méthode décrite par Milburn et Peroutka (*J. Neurochem.*, **52**, 1787-1792, 1989).
On utilise des rats mâles Sprague-Dawley, de 150 à 200 g, dans tous les essais. On en prélève le cortex cérébral et on l'homogénéise dans 20 volumes (poids/volume) de tampon Hepes 25 mM ou de tampon Hepes 25 mM contenant du chlorure de sodium (180 mM), du chlorure de calcium (2,5 mM), du chlorure de potassium (5 mM) et du chlorure de magnésium (1,2 mM) (pH=7,4), à l'aide d'un broyeur Polytron^{TM}. Après centrifugation de la suspension pendant 10mn à 45000×g, on remet le culot en suspension dans le volume initial de tampon, contenant éventuellement 0,05% de Triton X-100^{TM}, et on effectue une première incubation de 30mn à 37°C. On effectue ensuite encore deux centrifugations comme précédemment décrit, et on reprend le culot final dans 11,7 volumes de tampon Hepes 25 mM à pH=7,4.
On détermine la liaison de la [³H]quipazine (51,6-69,8 Ci/mmole, New England Nuclear, Boston, Ma, USA) en faisant incuber 150 µl de la suspension membranaire avec le radioligand (0,8 nM) dans un volume final de 1 ml, pendant 30mn à 25°C, en absence ou en présence du composé à étudier. L'incubation a lieu en présence de 0,1 µM de paroxétine et de 1 µM de kétansérine. La liaison non spécifique est déterminée en présence de 1 µM d'ondansetron. Après l'incubation, on dilue le mélange testé avec 5 ml de tampon Tris-HCl glacé 50 mM (pH=7,4 à 0°C). On collecte les membranes par filtration sur des filtres Whatman GF/B^{TM} prétraités avec 0,05% de polyéthylèneimine, et on les lave avec trois volumes de 5 ml de tampon Tris-HCl glacé 50 mM.
La radioactivité retenue sur les filtres est mesurée par spectrométrie de scintillation liquide à une efficacité de 50 à 60%.

Les résultats s'expriment par la concentration (CI₅₀) de composé étudié qui inhibe 50% de la liaison de la [³H]quipazine, déterminée par une méthode graphique ou mathématique. Les composés de l'invention les plus actifs dans cet essai se caractérisent par des CI₅₀ inférieures à 1 nM (10⁻⁹M).

Les composés de l'invention ont été également testés quant à leur effet sur le réflexe de Bezold-Jarisch, c'est-à-dire une intense bradycardie, provoqué par injection intraveineuse de sérotonine. Ce reflexe met en jeu la stimulation des récepteurs spécifiques 5-HT₃ du nerf vague, ce qui provoque une dépolarisation et donc une sécrétion d'acétylcholine, qui est le neurotransmetteur vagal naturel.
On anesthésie des rats mâles Sprague-Dawley à l'uréthane (1 à 25 g/kg par voie intrapéritonéale), on mesure la pression sanguine grâce à un cathéter placé dans l'artère carotide, et on se sert des impulsions de pression pour activer un cardiotachymètre. Des canules sont placées dans les deux veines fémorales pour faciliter l'administration intraveineuse des produits.
On trace les courbes doses/réponses de la bradycardie provoquée par injection de doses de 30 µg/kg de sérotonine, avant et après l'injection des composés à étudier.
Les composés de l'invention les plus actifs dans cet essai inhibent la bradycardie provoquée par la sérotonine d'au moins 50% à une dose de 10 µg/kg administrée par voie intraveineuse.

Un autre essai *in vivo* est celui de la vidange gastrique chez le rat, selon un protocole décrit par Scarpignato *(J. Pharmacol.,* (Paris) **14**(2), 261-268, 1983).
Les animaux sont des rats CD mâles de 180 à 200 g, à jeun depuis 24h. Les composés à étudier sont administrés par voie intrapéritonéale ou orale, 15 ou 30mn avant l'absorption d'un repas liquide non digestible (méthylcellulose et rouge de phénol). Les animaux sont sacrifiés 10mn après l'administration du repas, et la quantité de rouge de phénol restant dans l'estomac est dosée par spectrophotométrie, un groupe de rats témoins étant sacrifié immédiatement après le repas.
Les composés de l'invention les plus actifs dans cet essai augmentent la vidange gastrique après une dose administrée de 1 mg/kg par voie intrapéritonéale ou orale.

Les composés de l'invention ont également été étudiés quant à leurs effets sur l'émèse chez le furet (mâle, 1 à 1,4 kg), selon une méthode décrite par Costall *et al*. (*Neuropharmacology* **25**(8), 959-961, 1986).
Les composés à étudier ou du sérum physiologique sont administrés par voie intraveineuse (veine jugulaire), sous anesthésie à l'halothane, immédiatement avant une perfusion intraveineuse de cisplatine (10 mg/kg en 10mn).
On observe ensuite les animaux pendant 3h, en notant le nombre de crises émétiques, le nombre total de spasmes et de vomissements, ainsi que le délai d'apparition de la première crise.
Les composés de l'invention les plus actifs dans cet essai montrent un effet anti-émétique après administration d'une dose inférieure à 5 mg/kg par voie intraveineuse.

Enfin les composés de l'invention ont été étudiés quant à leurs effets sur les récepteurs 5-HT atypiques (5-HT₄) dans l'iléon de cobaye, d'après Craig et Clarke, *J. Pharm. Exp. Ther.*, **252** (3), 1378-1386, (1990).
Des cobayes tricolores Jegard mâles de 300 à 400 g sont assommés et saignés. On prélève rapidement un fragment d'iléon de 3 cm environ, au niveau de la jonction iléocoecale, et on le lave avec 10 ml de tampon Krebs tiédi (composition en mM : NaCl=118 ; CaCl₂=2,6 ; KCl=4,9 ; NaH₂PO₄=1 ; MgSO₄=1,2 ; NaHCO₃=25 ; Glucose=11,7). L'iléon est monté sur une pipette de 2 ml et le muscle longitudinal est séparé soigneusement avec un coton dentaire imbibé de tampon Krebs. L'organe est connecté à un transducteur isométrique sous une tension basale de 0,5 g et maintenu dans un bain de Krebs à 37°C aéré par un courant carbogène. Après un repos d'environ 30mn on applique une stimulation électrique (0,2 Hz ; 1,5 ms ; tension supramaximale ≤45 V) grâce à des électrodes de champ H. Sachs, modèle F2H reliées à un stimulateur Grass S88^{TM}, jusqu'à stabilisation des contractions (ou "twitches"). On ajoute alors dans le bain 3·10⁻⁷M de phénoxybenzamine, ce qui diminue l'amplitude des contractions jusqu'à environ 50% (≤30mn). L'organe est ensuite lavé six fois à 5mn d'intervalle. Avant le quatrième lavage on ajoute de la sérotonine (3·10⁻⁷M). Si nécessaire on diminue l'amplitude des contractions à 50% de l'amplitude supramaximale par diminution de la tension électrique après le sixième lavage. On construit une courbe concentration-effet par additions cumulatives, à 1mn d'intervalle, du composé à étudier.
Les réponses sont mesurées en terme de capacité à restaurer l'amplitude des contractions au niveau de celle obtenue grâce à la tension supramaximale et après traitement avec la phénoxybenzamine.
Les composés de l'invention se comportent comme des agonistes, agonistes partiels ou antagonistes desdits récepteurs, certains d'entre eux étant actifs à des concentrations inférieures à 10 nM.

Les résultats des essais biologiques montrent que les composés de l'invention sont des ligands des récepteurs sérotoninergiques. Comme montré ci-dessus, ils ont en particulier une interaction avec les récepteurs de types 5-HT₃ et 5-HT₄.
Ils peuvent donc être utilisés pour le traitement et la prévention des désordres dans lesquels les récepteurs 5-HT sont impliqués, tels que nausées et vomissements, par exemple consécutifs à un traitement antitumoral ou à l'administration d'un anesthésique ; troubles du système nerveux central tels que la schizophrénie, la manie, l'anxiété et la dépression ; troubles de la cognition tels que la démence sénile ou présénile d'Alzheimer ; dyskinésie, douleurs, migraines et maux de tête ; troubles de la dépendance ou du sevrage d'alcool ou de drogues ; troubles de la fonction gastrointestinale tels que dyspepsie, ulcère peptique, aigreurs d'estomac, flatulences ; troubles du système cardiovasculaire et troubles respiratoires.

A cet effet ils peuvent être présentés sous toutes formes appropriées à l'administration orale ou parentérale, telles que comprimés, dragées, gélules, capsules, suspensions ou solutions buvables ou injectables, etc. en association avec des excipients convenables, et dosées pour permettre une administration de 0,005 à 5 mg/kg de 1 à 4 fois par jour.

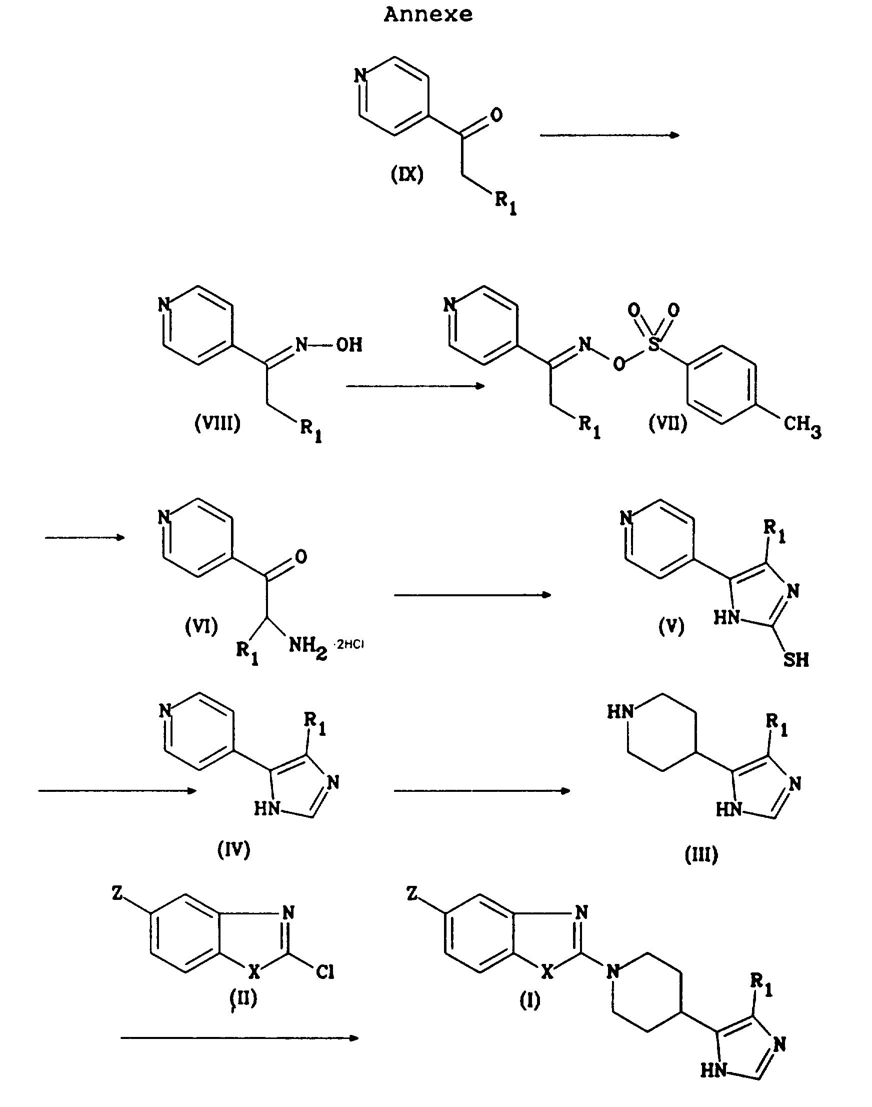

## Revendications (Revendications pour l'(les) Etat(s) contractant(s) suivant(s): AT, BE, CH, DK, DE, FR, GB, IT, LI, LU, MC, NL, PT, SE)

1. Composés répondant à la formule générale (I) 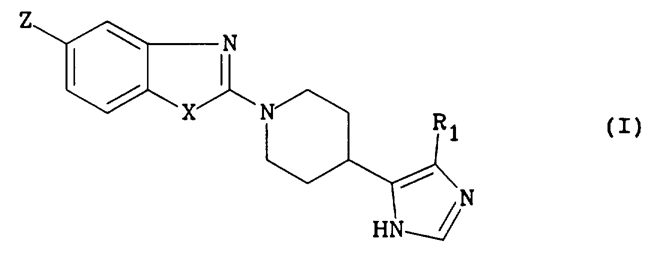 dans laquelle
R₁ représente soit un atome d'hydrogène, soit un groupe (C₁₋₆)alkyle droit ou ramifié, soit un groupe cyclo(C₃₋₈)alkyle,
X représente soit un atome d'oxygène, soit un atome de soufre soit un groupe de formule générale N-R₃ dans laquelle R₃ est un atome d'hydrogène, un groupe (C₁₋₈)alkyle droit ou ramifié, cyclo(C₃₋₆)alkyle, cyclo(C₃₋₆)alkylméthyle, (C₁₋₄)alcoxy(C₁₋₄)alkyle, phényle, pyridin-4-yle, pyridin-3-yle,
Z représente un atome d'hydrogène ou de fluor ainsi que leurs sels d'addition à des acides pharmaceutiquement acceptables.

2. Composés selon la revendication 1, caractérisés en ce que
R₁ représente soit un atome d'hydrogène, soit un groupe (C₁₋₆)alkyle droit ou ramifié,
X représente un groupe de formule générale N-R₃ dans laquelle R₃ est un groupe (C₁₋₈)alkyle droit ou ramifié,
Z représente un atome d'hydrogène.

3. Le 2-[4-(5-Méthyl-1*H*-imidazol-4-yl)pipéridin-1-yl]-1-(1-méthyléthyl)-1*H*-benzimidazole et ses sels d'addition à des acides pharmaceutiquement acceptables.

4. Le 2-[4-(1*H*-imidazol-4-yl)pipéridin-1-yl]-1-(1-méthyléthyl)-1*H*-benzimidazole et ses sels d'addition à des acides pharmaceutiquement acceptables.

5. Procédé de préparation des composés selon la revendication 1, procédé caractérisé en ce que l'on fait réagir un composé de formule générale (IX) 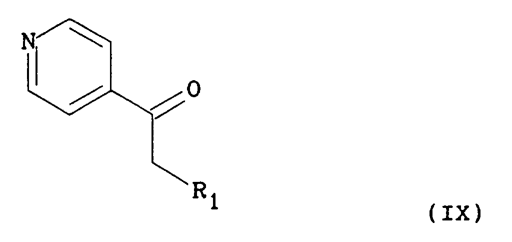 dans laquelle R₁ est tel que défini dans la revendication 1, avec du chlorhydrate d'hydroxylamine, pour obtenir un composé de formule générale (VIII) 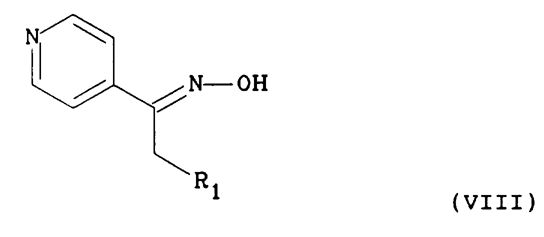 dans laquelle R₁ est tel que défini dans la revendication 1, composé que l'on fait réagir avec du chlorure de tosyle pour obtenir un composé de formule générale (VII) 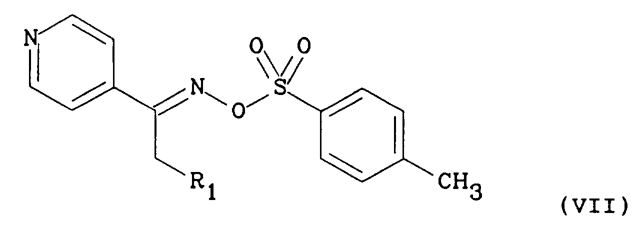 puis on ajoute d'abord une solution d'éthylate de potassium dans l'éthanol absolu, puis de l'acide chlorhydrique concentré, pour obtenir un composé de formule générale (VI) 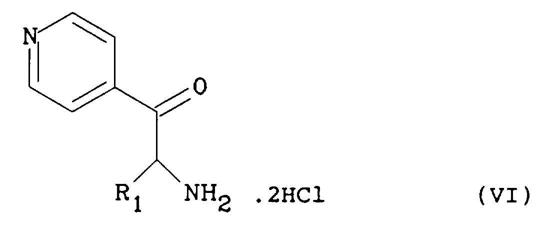 dans laquelle R₁ est tel que défini dans la revendication 1, composé auquel on additionne du thiocyanate de potassium pour obtenir un composé de formule générale (V) 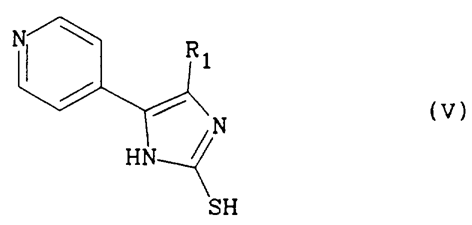 dans laquelle R₁ est tel que défini dans la revendication 1, puis on ajoute de l'acide nitrique et l'on obtient un composé de formule générale (IV) 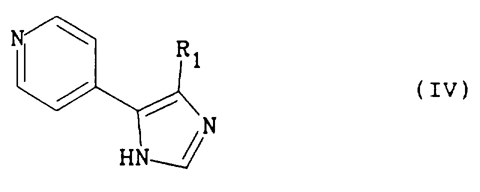 dans laquelle R₁ est tel que défini dans la revendication 1, puis on effectue une hydrogénation catalytique, pour obtenir un composé de formule générale (III) 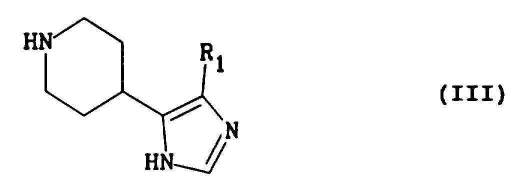 dans laquelle R₁ est tel que défini dans la revendication 1, composé que l'on fait réagir avec un composé de formule générale (II)

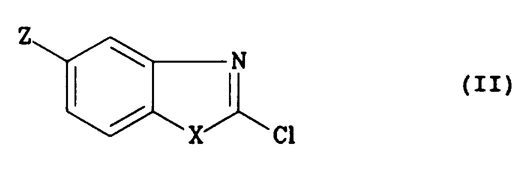

6. Médicament caractérisé en ce qu'il contient un composé selon l'une quelconque des revendications 1 à 4.

7. Composition pharmaceutique, caractérisée en ce qu'elle contient un composé selon l'une quelconque des revendications 1 à 4, associé à tout excipient pharmaceutiquement acceptable.

## Revendications (Revendications pour l'(les) Etat(s) contractant(s) suivant(s): ES, GR)

1. Procédé de préparation des composés répondant à la formule générale (I) 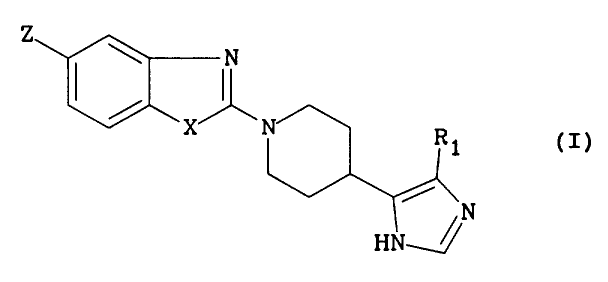 dans laquelle
R₁ représente soit un atome d'hydrogène, soit un groupe (C₁₋₆)alkyle droit ou ramifié, soit un groupe cyclo(C₃₋₈)alkyle,
X représente soit un atome d'oxygène, soit un atome de soufre soit un groupe de formule générale N-R₃ dans laquelle R₃ est un atome d'hydrogène, un groupe (C₁₋₈)alkyle droit ou ramifié, cyclo(C₃₋₆)alkyle, cyclo(C₃₋₆)alkylméthyle, (C₁₋₄)alcoxy(C₁₋₄)alkyle, phényle, pyridin-4-yle, pyridin-3-yle,
Z représente un atome d'hydrogène ou de fluor ainsi que de leurs sels d'addition à des acides pharmaceutiquement acceptables, procédé caractérisé en ce que l'on fait réagir un composé de formule générale (IX) 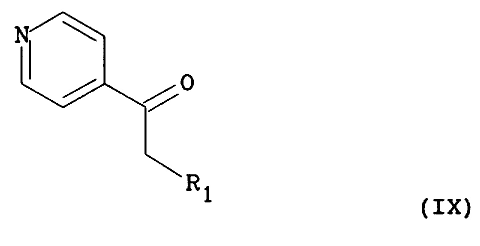
dans laquelle R₁ est tel que défini ci-dessus avec du chlorhydrate d'hydroxylamine, pour obtenir un composé de formule générale (VIII) 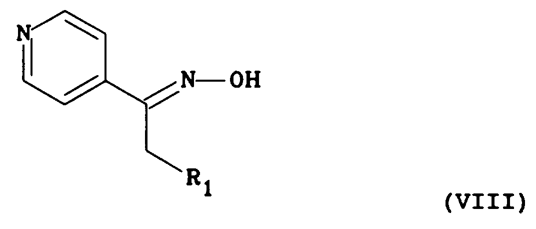 dans laquelle R₁ est tel que défini ci-dessus,
composé que l'on fait réagir avec du chlorure de tosyle pour obtenir un composé de formule générale (VII) 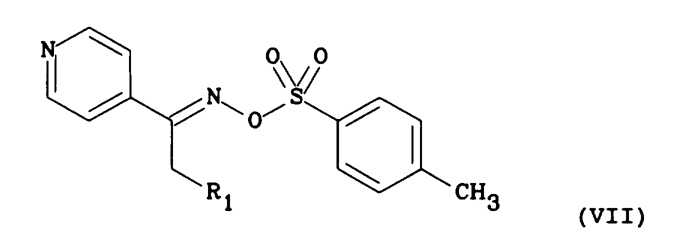 puis on ajoute d'abord une solution d'éthylate de potassium dans l'éthanol absolu, puis de l'acide chlorhydrique concentré, pour obtenir un composé de formule générale (VI) 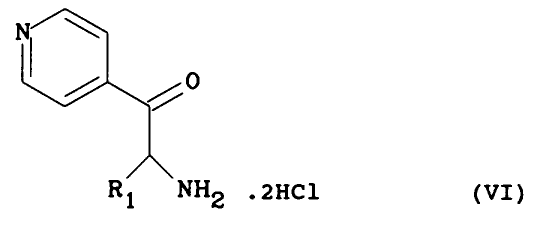 dans laquelle R₁ est tel que défini ci-dessus,
composé auquel on additionne du thiocyanate de potassium pour obtenir un composé de formule générale (V) 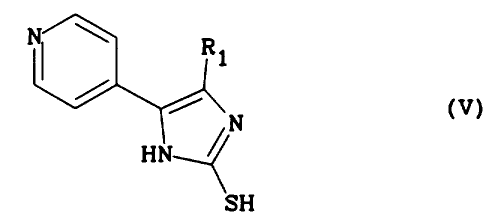 dans laquelle R₁ est tel que défini ci-dessus,
puis on ajoute de l'acide nitrique et l'on obtient un composé de formule générale (IV) 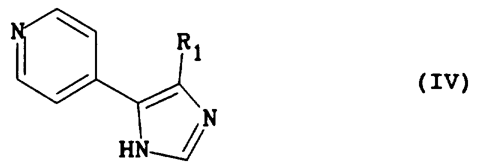 dans laquelle R₁ est tel que défini ci-dessus,
puis on effectue une hydrogénation catalytique, pour obtenir un composé de formule générale (III) 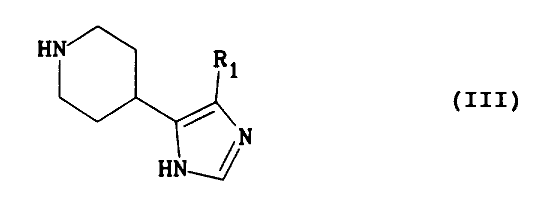 dans laquelle R₁ est tel que défini ci-dessus, composé que l'on fait réagir avec un composé de formule générale (II)

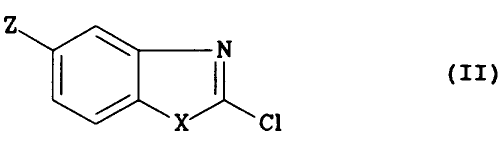

## Patentansprüche (Patentansprüche für folgende(n) Vertragsstaat(en): AT, BE, CH, DK, DE, FR, GB, IT, LI, LU, MC, PT, SE)

1. Verbindungen der allgemeinen Formel (I) 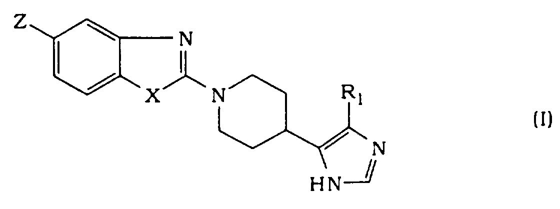 in der
R₁ entweder ein Wasserstoffatom oder eine geradkettige oder verzweigte (C₁₋₆)-Alkylgruppe oder eine Cyclo-(C₃₋₈)-alkylgruppe,
X entweder ein Sauerstoffatom oder ein Schwefelatom oder eine Gruppe der allgemeinen Formel N-R₃, in der R₃ ein Wasserstoffatom, eine geradkettige oder verzweigte (C₁₋₈)-Alkylgruppe, eine Cyclo-(C₃₋₆)-alkylgruppe, eine Cyclo-(C₃₋₆)-alkylmethylgruppe, eine (C₁₋₄)-Alkoxy-(C₁₋₄)-alkylgruppe, eine Phenylgruppe, eine Pyridin-4-yl-gruppe oder eine Pyridin-3-yl-gruppe darstellt, und
Z ein Wasserstoffatom oder ein Fluoratom bedeuten,
sowie deren Additionssalze mit pharmazeutisch annehmbaren Säuren.

2. Verbindungen nach Anspruch 1, **dadurch gekennzeichnet**, daß R₁ entweder ein Wasserstoffatom oder eine geradkettige oder verzweigte (C₁₋₆)-Alkylgruppe,
X eine Gruppe der allgemeinen Formel N-R₃, in der R₃ eine geradkettige oder verzweigte (C₁₋₈)-Alkylgruppe darstellt, und
Z ein Wasserstoffatom bedeuten.

3. 2-[4-(5-Methyl-1H-imidazol-4-yl)-piperidin-1-yl]-1-(1-methylethyl)-1H-benzimidazol und dessen Additionssalze mit pharmazeutisch annehmbaren Säuren.

4. 2-[4-(1H-Imidazol-4-yl)-piperidin-1-yl]-1-(1-methylethyl)-1H-benzimidazol und dessen Additionssalze mit pharmazeutisch annehmbaren Säuren.

5. Verfahren zur Herstellung der Verbindungen nach Anspruch 1, **dadurch** **gekennzeichnet**, daß man eine Verbindung der allgemeinen Formel (IX) 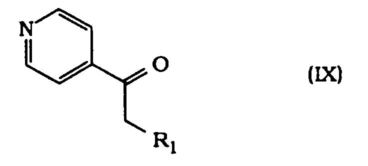 in der R₁ die in Anspruch 1 angegebenen Bedeutungen besitzt, mit Hydroxylamin-Hydrochlorid umsetzt zur Bildung eines Verbindung der allgemeinen Formel (VIII) 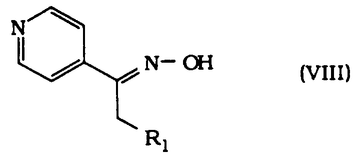 in der R₁ die in Anspruch 1 angegebenen Bedeutungen besitzt, welche Verbindung man mit Tosylchlorid umsetzt zur Bildung einer Verbindung der allgemeinen Formel (VII) 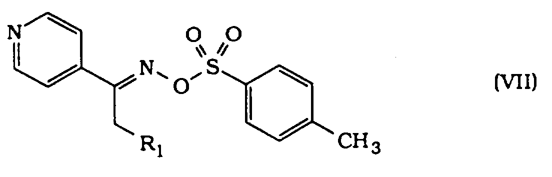 wonach man zunächst eine Lösung von Kaliumethylat in absolutem Ethanol und dann konzentrierte Chlorwasserstoffsäure zugibt zur Bildung einer Verbindung der allgemeinen Formel (VI) 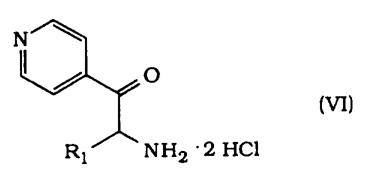 in der R₁ die in Anspruch 1 angegebenen Bedeutungen besitzt, welche Verbindung man mit Kaliumthiocyanat versetzt zur Bildung einer Verbindung der allgemeinen Formel (V) 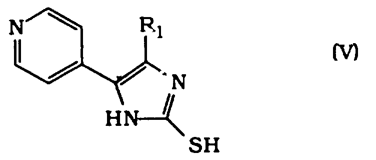 in der R₁ die in Anspruch 1 angegebenen Bedeutungen besitzt, worauf man Salpetersäure zugibt und eine Verbindung der allgemeinen Formel (IV) 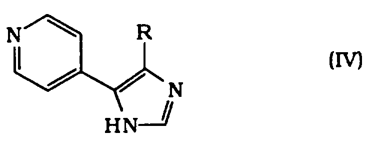 erhält, in der R₁ die in Anspruch 1 angegebenen Bedeutungen besitzt, wonach man eine katalytische Hydrierung durchführt zur Bildung einer Verbindung der allgemeinen Formel (III) 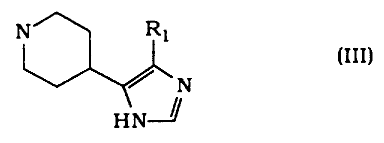 in der R₁ die in Anspruch 1 angegebenen Bedeutungen besitzt, welche Verbindung man mit einer Verbindung der allgemeinen Formel (II) 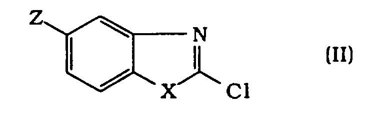 umsetzt.

6. Arzneimittel, **dadurch gekennzeichnet**, daß es eine Verbindung nach einem der Ansprüche 1 bis 4 enthält.

7. Pharmazeutische Zubereitung, **dadurch gekennzeichnet**, daß sie eine Verbindung nach einem der Ansprüche 1 bis 4 in Kombination mit irgendeinem geeigneten pharmazeutischen Trägermaterial umfaßt.

## Patentansprüche (Patentansprüche für folgende(n) Vertragsstaat(en): ES, GR)

1. Verfahren zur Herstellung der Verbindungen der allgemeinen Formel (I) 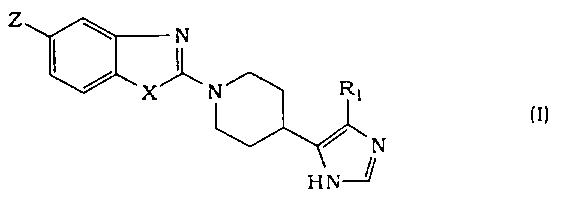 in der
R₁ entweder ein Wasserstoffatom oder eine geradkettige oder verzweigte (C₁₋₆)-Alkylgruppe oder eine Cyclo-(C₃₋₈)-alkylgruppe,
X entweder ein Sauerstoffatom oder ein Schwefelatom oder eine Gruppe der allgemeinen Formel N-R₃, in der R₃ ein Wasserstoffatom, eine geradkettige oder verzweigte (C₁₋₈)-Alkylgruppe, eine Cyclo-(C₃₋₆)-alkylgruppe, eine Cyclo-(C₃₋₆)-alkylmethylgruppe, eine (C₁₋₄)-Alkoxy-(C₁₋₄)-alkylgruppe, eine Phenylgruppe, eine Pyridin-4-yl-gruppe oder eine Pyridin-3-yl-gruppe darstellt, und
Z ein Wasserstoffatom oder ein Fluoratom bedeuten,
sowie von deren Additionssalzen mit pharmazeutisch annehmbaren Säuren, **dadurch gekennzeichnet**, daß man eine Verbindung der allgemeinen Formel (IX) 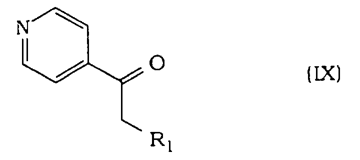 in der R₁ die oben angegebenen Bedeutungen besitzt, mit Hydroxylamin-Hydrochlorid umsetzt zur Bildung eines Verbindung der allgemeinen Formel (VIII) 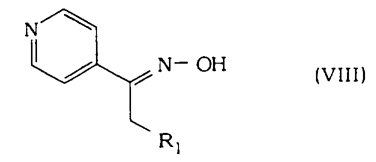 in der R₁ die oben angegebenen Bedeutungen besitzt, welche Verbindung man mit Tosylchlorid umsetzt zur Bildung einer Verbindung der allgemeinen Formel (VII) 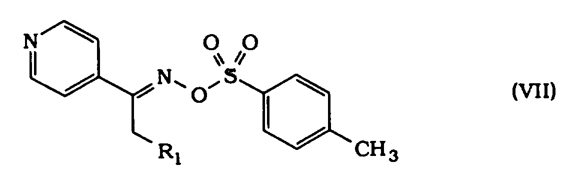 wonach man zunächst eine Lösung von Kaliumethylat in absolutem Ethanol und dann konzentrierte Chlorwasserstoffsäure zugibt zur Bildung einer Verbindung der allgemeinen Formel (VI) 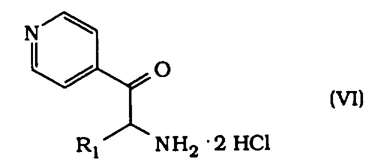 in der R₁ die oben angegebenen Bedeutungen besitzt, welche Verbindung man mit Kaliumthiocyanat versetzt zur Bildung einer Verbindung der allgemeinen Formel (V) 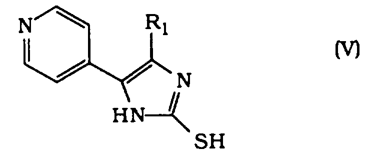 in der R₁ die oben angegebenen Bedeutungen besitzt, worauf man Salpetersäure zugibt und eine Verbindung der allgemeinen Formel (IV) 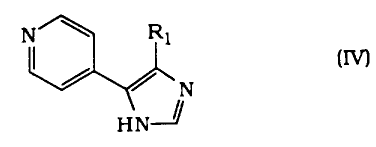 erhält, in der R₁ die oben angegebenen Bedeutungen besitzt, wonach man eine katalytische Hydrierung durchführt zur Bildung einer Verbindung der allgemeinen Formel (III) 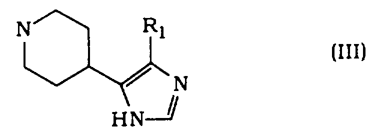 in der R₁ die oben angegebenen Bedeutungen besitzt, welche Verbindung man mit einer Verbindung der allgemeinen Formel (II) 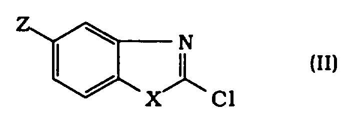 umsetzt.

## Claims (Claims for the following Contracting State(s): AT, BE, CH, DK, DE, FR, GB, IT, LI, LU, MC, NL, PT, SE)

1. Compounds corresponding to the general formula (I) 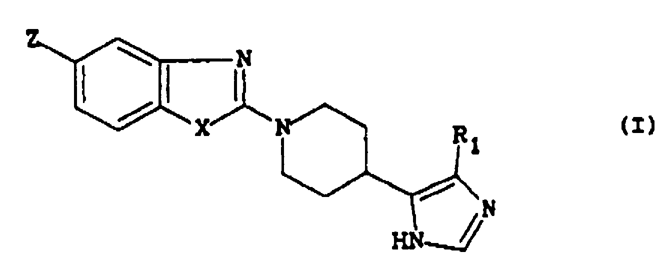 in which
R₁ represents either a hydrogen atom, or a linear or branched (C₁₋₆)alkyl group, or a cyclo(C₃₋₈)alkyl group,
X represents either an oxygen atom, or a sulphur atom or a group of general formula N-R₃ in which R₃ is a hydrogen atom, a linear or branched (C₁₋₈)alkyl group, a cyclo(C₃₋ ₆)alkyl, cyclo(C₃₋₆)alkylmethyl, (C₁₋₄)alkoxy-(C₁₋₄)alkyl, phenyl, pyridin-4-yl, pyridin-3-yl group,
Z represents a hydrogen or a fluorine atom as well as their addition salts with pharmaceutically acceptable acids.

2. Compounds according to Claim 1, characterised in that R₁ represents either a hydrogen atom or a linear or branched (C₁₋₆)alkyl group,
X represents a group of general formula N-R₃ in which R₃ is a linear or branched (C₁₋₈)alkyl group,
Z represents a hydrogen atom.

3. 2-[4-(5-Methyl-1*H*-imidazol-4-yl)piperidin-1-yl]-1-(1-methylethyl)-1*H*-benzimidazole and its addition salts with pharmaceutically acceptable acids.

4. 2-[4-(1*H*-imidazol-4-yl)piperidin-1-yl]-1-(1-methylethyl)-1*H*-benzimidazole and its addition salts with pharmaceutically acceptable acids.

5. Method of preparing the compounds according to Claim 1, method characterised in that a compound of general formula (IX) 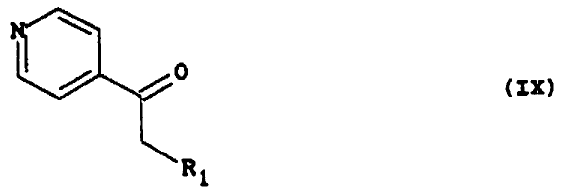 in which R₁ is as defined in Claim 1, in reacted with hydroxylamine hydrochloride in order to obtain a compound of general formula (VIII) 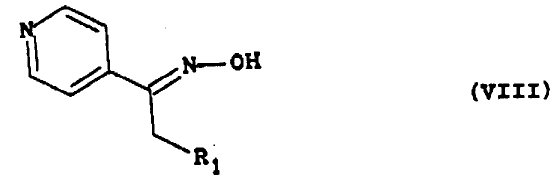 in which R₁ is as defined in Claim 1, compound which is reacted with tosyl chloride in order to obtain a compound of general formula (VII) 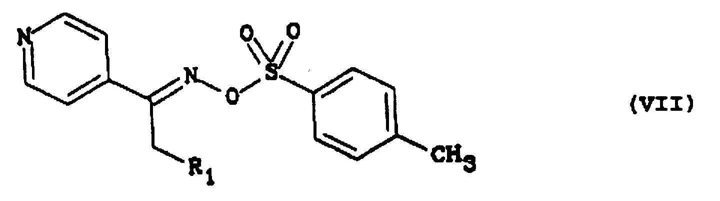 and then a solution of potassium ethylate in absolute ethanol is first added, and then concentrated hydrochloric acid, in order to obtain a compound of general formula (VI) 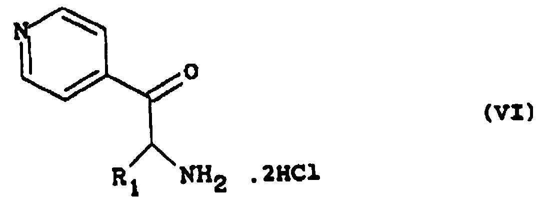 in which R₁ is as defined in Claim 1, compound to which potassium thiocyanate is added in order to obtain a compound of general formula (V) 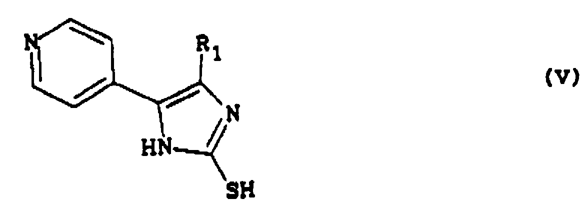 in which R₁ is as defined in Claim 1, and then nitric acid is added and a compound of general formula (IV) 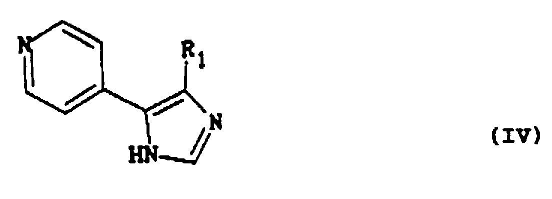 is obtained, in which R₁ is as defined in Claim 1, and then a catalytic hydrogenation is carried out in order to obtain a compound of general fomula (III) 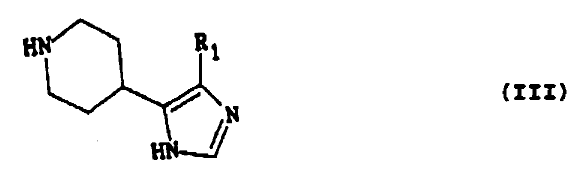 in which R₁ is as defined in Claim 1, compound which is reacted with a compound of general formula (II)

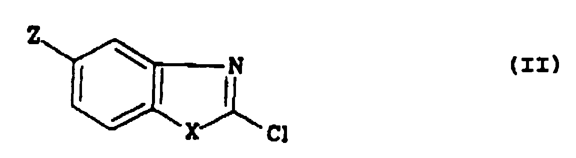

6. Medicinal product characterised in that it contains a compound according to any one of Claims 1 to 4.

7. Pharmaceutical composition characterised in that it contains a compound according to any one of Claims 1 to 4, combined with any pharmaceutically acceptable excipient.

## Claims (Claims for the following Contracting State(s): ES, GR)

1. Compounds corresponding to the general formula (I) 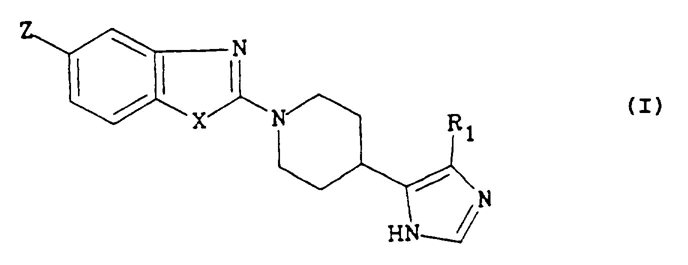 in which
R₁ represents either a hydrogen atom, or a linear or branched (C₁₋₆)alkyl group, or a cyclo(C₃₋₈)alkyl group,
X represents either an oxygen atom, or a sulphur atom or a group of general formula N-R₃ in which R₃ is a hydrogen atom, a linear or branched (C₁₋₈)alkyl group, a cyclo(C₃₋ ₆)alkyl, cyclo(C₃₋₆)alkylmethyl, (C₁₋₄)alkoxy-(C₁₋₄)alkyl, phenyl, pyridin-4-yl, pyridin-3-yl group,
Z represents a hydrogen or a fluorine atom as well as their addition salts with pharmaceutically acceptable acids, method characterised in that a compound of general formula (IX) 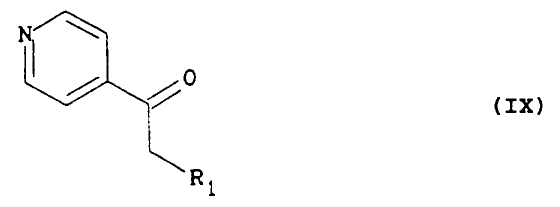
in which R₁ is as defined above, is reacted with hydroxylamine hydrochloride in order to obtain a compound of general formula (VIII) 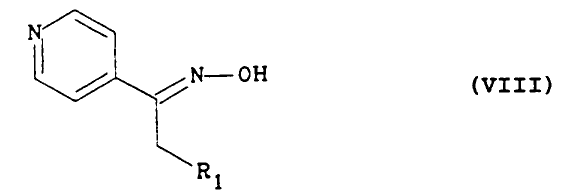 in which R₁ is as defined above , compound which is reacted with tosyl chloride in order to obtain a compound of general formula (VII) 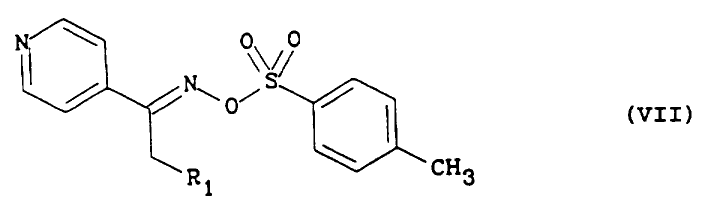 and then a solution of potassium ethylate in absolute ethanol is first added, and then concentrated hydrochloric acid, in order to obtain a compound of general formula (VI) 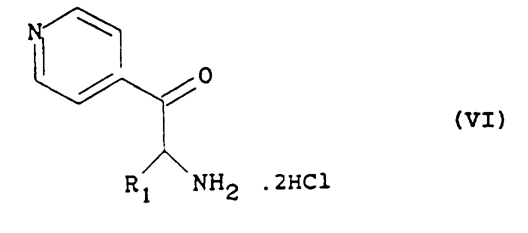 in which R₁ is as defined above, compound to which potassium thiocyanate is added in order to obtain a compound of general formula (V) 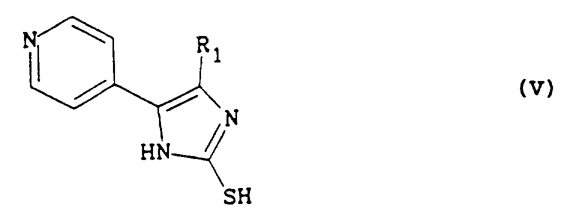 in which R₁ is as defined above, and then nitric acid is added and a compound of general formula (IV) 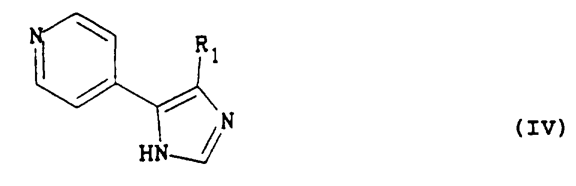 is obtained, in which R₁ is as defined above and then a catalytic hydrogenation is carried out in order to obtain a compound of general formula (III) 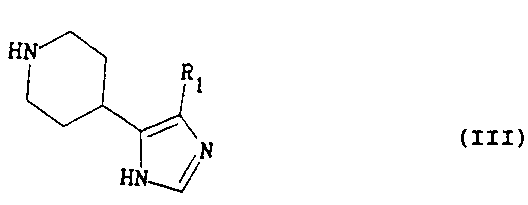 in which R₁ is as defined above, compound which is reacted with a compound of general formula (II)
